# EUROPÄISCHE PATENTSCHRIFT

(11) **EP 0 977 560 B1**
(45) Veröffentlichungstag und Bekanntmachung des Hinweises auf die Patenterteilung: **30.06.2004**
(21) Anmeldenummer: 98921471.3
(22) Anmeldetag: 20.04.1998
(51) Int. Cl.: A61K 31/19, A61K 31/70, A61P 29/00

(54) **IBUPROFENTHIOESTER ALS HEMMER DER Nf-kappa B ABHÄNGIGEN BILDUNG VON MEDIATOREN VON ENTZÜNDUNG UND SCHMERZ**
IBUPROFEN THIOESTERS AS INHIBITORS OF Nf-kappaB-DEPENDENT PAIN AND INFLAMMATION MEDIATOR FORMATION
THIOESTERS D'IBUPROFENE UTILISES COMME INHIBITEURS DE LA FORMATION DE MEDIATEURS D'INFLAMMATION ET DE DOULEUR Nf-kappa-B-DEPENDANTE

(30) Priorität: 21.04.1997 DE 19716713
(43) Veröffentlichungstag der Anmeldung: 09.02.2000
(73) Patentinhaber: PAZ Arzneimittel- Entwicklungsgesellschaft mbH, 65933 Frankfurt am Main (DE)
(72) Erfinder: BANG, Holger, 06120 Halle (DE); BRUNE, Kay, Prof.Dr.Dr., D-91080 Rathsberg (DE); GEISSLINGER, Gerd, D-91085 Weisendorf (DE); PAHL, Andreas, D-91054 Erlangen (DE); SCHEUREN, Nicole, D-91052 Erlangen (DE); NEUPERT, Werner, D-91056 Erlangen (DE)
(74) Vertreter: Böhm, Brigitte, Dipl.-Chem. Dr.
(86) Internationale Anmeldenummer: PCT/EP1998/002318
(87) Internationale Veröffentlichungsnummer: WO 1998/047502

(56) Entgegenhaltungen:
- EP-A- 0 255 164
- EP-A- 0 299 558
- WO-A-93/10762
- WO-A-97/48391
- W. NEUPERT ET AL.: "STEREOSELECTIVE INHIBITION OF CYCLOOXYGENASE-1 (COX-1) AND COX-2 BY IBOPROFENOYL-CoA-THIOESTERS AND IBUPROFEN ENANTIOMERS" NAUNYN SCHMIEDEBERG'S ARCH. PHARMACOL., Bd. 335, Nr. sUPPL., 11. - 13. März 1997, Seite R127 XP002077651
- W.NEUPERT ET AL.: "Effects of ibuprofen enantiomers and its coenzyme A thioesters on human prostaglandin endoperoxide synthases" BR.J.PHARMACOL., Bd. 122, Nr. 3, Oktober 1997, Seiten 487-492, XP002077652
- G.KLEIN: "Isomerischer Ballast: Pharmakologische Spitzfindigkeit oder medizinischer Fortschritt?" THERAPIEWOCHE, Bd. 8, Nr. 12, Dezember 1993, Seiten 652-657, XP002077653 ÖSTERREICH
- C.-S. CHEN ET AL.: "Metabolic stereoisomeric inversion of ibuprofen in mammals" BIOCHIM. BIOPHYS. ACTA, Bd. 1078, Nr. 3, 1991, Seiten 411-417, XP002077654
- R.D.KNIHINICKI ET AL.: "CHIRAL INVERSION OF 2-ARYLPROPIONIC ACID NON-STEROIDAL ANTI-INFLAMMATORY DRUGS--1. IN VITRO STUDIES OF IBUPROFEN AND FLURBIPROFEN" BIOCHEM.PHARMACOL., Bd. 38, Nr. 24, 1989, Seiten 4389-4395, XP002077655
- T.S.TRACY ET AL.: "METABOLIC INVERSION OF (R)-IBUPROFEN" DRUG METAB. DISPOS., Bd. 21, Nr. 1, Januar 1993 - Februar 1993, Seiten 114-120, XP002077656
- T.S.TRACY ET AL.: "METABOLIC INVERSION OF (R)-IBUPROFEN" DRUG.METAB.DISPOS., Bd. 20, Nr. 2, März 1992 - April 1992, Seiten 322-327, XP002077657
- R.D.KNIHINICKI ET AL.: "CHIRAL INVERSION OF 2-ARYLPROPIONIC ACID NON-STEROIDAL ANTI-INFLAMMATORY DRUGS--II. RACEMIZATION AND HYDROLYSIS OF (R)- AND (S)-IBUPROFEN-CoA THIOESTERS" BIOCHEM.PHARMACOL., Bd. 42, Nr. 10, 24. Oktober 1991, Seiten 1905-1911, XP002077658
- S.D.HALL ET AL.: "The role of coenzyme A in the biotransformation of 2-arylpropionic acids" CHEM. BIOL. INTERACT., Bd. 90, Nr. 3, März 1994, Seiten 235-251, XP002077659
- S.MENZEL ET AL.: "Is the formation of R-ibuprofenyl-adenylate the first stereoselective step of chiral inversion?" BIOCHEM.PHARMACOL., Bd. 48, Nr. 5, 30. August 1994, Seiten 1056-1058, XP002077660
- C.KEMAL ET AL.: "COENZYME A ESTERS OF 2-ARYLOXYPHENOXYPROPIONATE HERBICIDES AND 2-ARYLPROPIONATE ANTIINFLAMMATORY DRUGS ARE POTENT AND STEREOSELECTIVE INHIBITORS OF RAT LIVER ACETYL-CoA CARBOXYLASE" LIFE SCI., Bd. 50, Nr. 7, 1992, Seiten 533-540, XP002077661
- A.M.EVANS: "Enantioselective pharmacodynamics and pharmacokinetics of chiral non-steroidal anti-inflammatory drugs" EUR.J.CLIN.PHARMACOL., Bd. 42, Nr. 3, März 1992, Seiten 237-256, XP002077662
- K.WILLIAMS ET AL.: "The stereoselective uptake of ibuprofen enantiomers into adipose tissue" BIOCHEM. PHARMACOL., Bd. 35, Nr. 19, 1986, Seiten 3403-3405, XP002077663
- K.M.KNIGHTS ET AL.: "Enhanced chiral inversion of R-ibuprofen in liver from rats treated with clofibric acid" BIOCHEM. PHARMACOL., Bd. 41, Nr. 11, 1991, Seiten 1775-1777, XP002077664
- R.BRUGGER ET AL.: "Isolation and Characterization of Rat Liver Microsomal R-Ibuprofenyl-CoA Synthetase" BIOCHEM.PHARMACOL., Bd. 52, Nr. 7, 1996, Seiten 1007-1013, XP002077665

## Beschreibung

(CoA-Thioester von Arylpropionsäuren, Arylessigsäuren und Salicylaten als Hemmer der Nf-κB abhängigen Bildung von Mediatoren von Entzündung und Schmerz)

Gegenstand der vorliegenden Erfindung sind Arzneimittel, die spezifisch und selektiv die Aktivierung des Transkriptionsfaktors Nf-KB durch entzündliche Stimuli blockieren und damit selektiv in die Entwicklung der Symptome von Entzündung und Schmerz eingreifen.

Schmerz- und Rheumamittel sind die am meisten verwendeten Arzneistoffe weltweit. Sie bestehen im wesentlichen aus Substanzen, die die Enzyme Cyclooxygenase 1 und 2 (PGHS-1 und PGHS-2) hemmen (Frölich, TIBS, Januar 1997 (Vol. 18), S. 30)). Dadurch wird die Bildung von bestimmten Entzündungsmediatoren, den Prostaglandinen, unterdrückt und die Entstehung und Perpetuation von Entzündungssymptomen wie Rötung, Schwellung, Erwärmung, Schmerz und eingeschränkte Funktion blockiert (Vane and Botting (1996) Overview - mechanism of action of anti-inflammatory drugs. In: Improved non-steroidal anti-inflammatory drugs - COX-2 enzyme inhibitors. Ed.: Vane J.R., Botting J., Botting R., S. 1-27, Lancester: Kluwer Academic Publishers). Dabei spielt es keine Rolle, ob die Entzündung auf der Basis von Verletzungen (Traumen), Infektionen (Bakterien, Viren, Pilze), Tumoren oder immunologischen Reaktionen (allergische Reaktionen, Autoimmunerkrankungen) entsteht. Neuerdings wird ein weiterer spezifischerer Therapieansatz versucht. Dabei soll der durch Entzündungsreize wie Phorbolester oder Zytokine im Entzündungsgewebe erfolgende, zur Bildung unterschiedlicher Entzündungsmediatoren führende Aktivierungsprozess über die Hemmung des zentalen Transkripionsfaktors Nf-κB unterbrochen werden (Bauerle and Henkel, Annu. Rev. Immunol. (1994) 12, S. 141-179; Barnes and Adcock, TiPS, Februar 1997 (Vol. 18), S. 46).

Die bisher verwendeten Inhibitoren der Produktion von Prostaglandinen, wie z.B. razemisches Ibuprofen, weisen alle unerwünschte Arzneimittelwirkungen auf, die z.T. auf der Hemmung der Prostaglandinproduktion durch die Cyclooxygenase 1 beruhen. Einige Organsysteme, wie z.B. die Magen-Darmschleimhaut, das Nierengewebe, die Lungenschleimhaut und Blutzellen bedürfen nämlich der andauernden Produktion von Prostaglandinen durch die konstitutiv vorhandene Cyclooxygenase 1. Bei Hemmung dieses Enzymsystems kommt es daher zu Schäden (Vane and Botting, supra). Aufgabe der vorliegenden Erfindung war es daher, Substanzen zu finden, die nur oder überwiegend die Prostaglandinsynthese in Zusammenhang mit Entzündungsprozessen, d.h. besonders im entzündeten Gewebe verhindern, die Produktion in allen anderen Geweben aber nicht oder nur geringfügig tangieren. Solche Arzneistoffe sollten z.B. nur oder überwiegend in den für die Entzündungssymptomatik entscheidenden Zellen enstehen oder aktiviert werden und nur die in diesen Zellen bei Entzündungen gebildete Cyclooxygenase 2 und zusätzlich andere Entzündungsmediatoren in ihrer Bildung hemmen.

Diese Aufgabe wird erfindungsgemäß gelöst durch ein Arzneimittel, enthaltend CoA-Thioester von Arylpropionsäuren, Arylessigsäuren oder Acetylsalicylaten oder R- oder/und S-ibuprofen-CoA-Thioester.

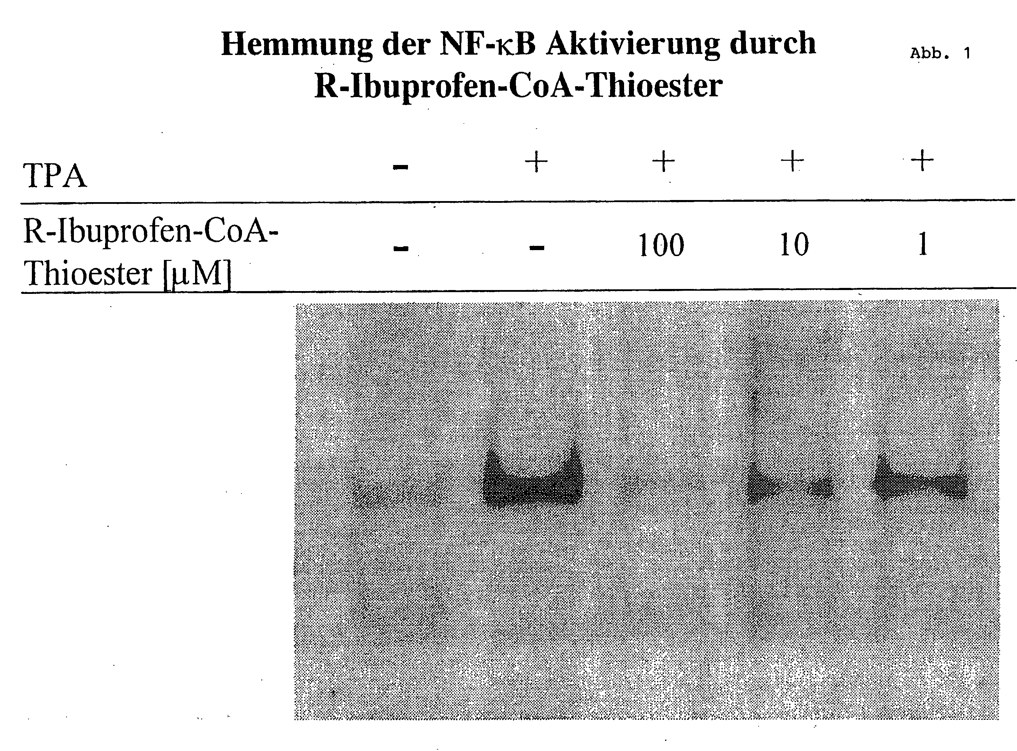

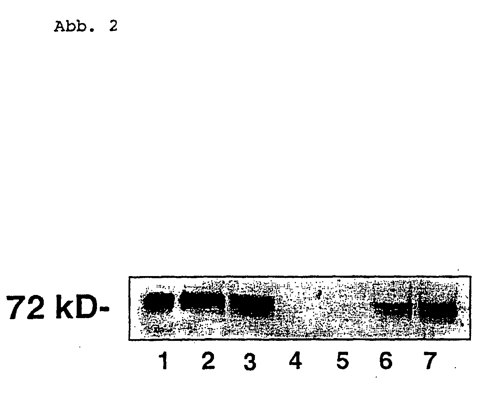

Bei der Analyse der Enantiomeren von Arylpropionsäuren, d.h. bekannter antientzündlicher Arzneistoffe, zeigte sich, daß das als unwirksamer betrachtete R-Enantiomer des Ibuprofens (Evans, Eur. J. Clin. Pharmacol. (1992) 42, 237-256; Klein, Therapiewoche Österreich; 8, Dezember 1993, Heft 12, 652-657) im Intermediärstoffwechsel zu R-und S-CoA-Thioestern metabolisiert wird (Menzel et al., Biochemical Pharmacology (1994), Vol. 48, Nr. 5, S. 1056-1058). Überraschenderweise erwiesen sich diese R-und S-Ibuprofen CoA-Thioester als potente und spezifische Hemmer der Aktivierung, des nukleären Transkriptionsfaktors Nf-κB (Abbildung 1). Als Ursache dieses überraschenden Befundes erwies sich, daß diese R-und S-CoA-Ibuprofenthioester zu einer Hemmung der Nf-κB-abhängigen Transkription führen (Abbildung 2). Da der nukleäre Transkriptionsfaktor Nf-κB für die Bildung einer Reihe von Eiweißstoffen (Zytokinen) und Enzymen (Cyclooxygenase 2) mit bekannt proinflammatorischen Eigenschaften verantwortlich ist (Barnes and Adcock, supra) erscheint der beobachtete neuartige Effekt der R-und S-Thioester von besonderer Bedeutung für die Hemmung, Verminderung und zeitliche Begrenzung von allen Symptomen akuter und chronischer Entzündungen. So ließ sich z.B. zeigen, daß die Induktion der Cyclooxygenase 2, die für die Bildung inflammatorischer Prostaglandine überwiegend verantwortlich ist ( Seibert et al., Novel Molecular Approaches to Anti-Inflammatory Theory, 1995, Birkhäuser Verlag, Basel, AAS 46, S. 41) in Entzündungszellen (Monocyten des Blutes) durch die genannten Thioester blockiert wurde, indem die genannten Thioester nicht nur die Aktivierung von Nf-κB hemmen, sondern auch die Bildung der Nf-κB abhängigen Cyclooxygenase blockieren (Abbildung 2).

Als Konsequenz dieser Erfindung ergibt sich, daß die genannten Thioester des R-Enantiomers des Ibuprofens hochaktive Hemmer der Aktivierung von Nf-κB durch entzündliche Stimuli und dadurch spezifische, antiphlogistische und analgetische Wirkstoffe sind. Sie hemmen besonders im entzündeten Gewebe die Bildung der Nf-κB abhängigen Entzündungsmediatoren.

Gleiches gilt für R-Ibuprofen selbst, welches im Körper zu den genannten Thioestern metabolisiert wird. Es können im Prinzip im Rahmen der Erfindung auch Razemate von Ibuprofen mit bis zu 49 % S-Ibuprofen verwendet werden, da hierbei die Wirkung des R-Ibuprofens immer noch die unerwünschten Wirkungen des S-Ibuprofens, welche in der Einleitung dargelegt wurden, überwiegt. Gleichfalls können Derivate der genannten Verbindungen verwendet werden, welche im Organismus in analoger Weise metabolisiert werden.

Außerdem liegen Anhaltspunkte dafür vor, daß dieselben Mechanismen auch mit Arylessigsäuren oder Acetylsalicylaten ablaufen. Diese Verbindungen sind daher im Rahmen der Erfindung mitumfaßt.

Die hier berichtete Beobachtung Widerspricht dem Stand der Technik, die bisher keinen eigenständigen pharmakologischen (antiphlogistischen oder analgetischen) Effekt für die Thioester des Ibuprofens oder des Prodrugs, R-Ibuprofen (A.M. Evans, Eur. J. Clin. Pharmacol. (1992) 42, S. 237-256; Klein, supra) vermutete. Dieser überraschende Befund führte zu der Erkenntnis, daß gerade diese Thioester, die entsprechenden Prodrugs und galenische Produkte vorteilhaft therapeutisch verwendet werden können, weil durch ihre Verwendung eine Verminderung der unerwünschten Arzneimittelwirkungen bei erhaltener Wirksamkeit zu erwarten ist. (Es wird ja nicht die für viele Organe wichtige Cyclooxygenase 1, s. oben, sondern unter anderem die im Entzündungsgewebe durch die entzündungsbedingte Aktivierung von Nf-κB entstehende Cyclooxygenase 2 an ihrer Entstehung gehindert.) Insbesondere die Ibuprofenthioester stellen somit ein neuartiges, bisher unbekanntes Wirkprinzip bei Entzündungen dar. Ihr Wirkungsspektrum ist vermutlich anders (breiter, vgl. z.B. A.S. Baldwin, Jr., Annu. Rev. Immunol. (1996) 14, 649-81) als dasjenige bekannter Hemmer von Cyclooxygenasen, da verschiedene Nf-κB abhängige Mediatoren vermindert gebildet werden. Ihr Nebenwirkungsspektrum ist vermutlich geringer, da keine Cyclooxygenase 1-Hemmung erfolgt.

Im Rahmen der Erfindung scheint es außerdem möglich, andere Nf-κB abhängigen Prozesse zu hemmen, die wiederum die Grundlage anderer Krankheiten oder unangenehmer Erscheinungen sind. Dies sind z.B. die Entstehung und das Wachstum von Tumoren, Autoimmunerkrankungen, allergische Reaktionen etc. Im Prinzip kann daher das erfindungsgemäße Arzneimittel für die Prophylaxe oder Behandlung,aller derartiger Erscheinungsformen angewandt werden, die auf Nf-κB abhänigen Prozessen beruhen.

### Beispiele für Wirkstoffe im Sinne dieses Patents

1. R- und S-Ibuprofen -(CoA)- Thioester und deren im gleichen Sinn aktiven Derivate wie Ester, Salz und andere übliche chemische Verbindungen etc.
2. Alle Prodrugs der Ibuprofenthioester wie z.B. R-Ibuprofen und seine Derivate soweit sie im Organismus zu CoA-Thioestern metabolisiert werden.
3. Alle pharmakologisch im gleichen Sinn wirksamen Präparationen, z.B. razemische Gemische von R-Ibuprofen mit bis zu 49% S-Ibuprofen

Die Abbildungen 1 und 2 zeigen, wie im Text bereits ausgeführt, die Grundlagen der vorliegenden Erfindung:

### Abbildung 1

Einfluß von R-Ibuprofen-CoA-Thioester auf die Aktivierung des Transkriptionsfaktors NF-κB in Jurkat-Zellen. Der Elektro Mobility-Shift Assay (DIG Gel Shift Kit, Boehringer Mannheim) zeigt, daß in Phorbolester (TPA) stimulierten Jurkat-Zellen eine zweistündige Preinkubation mit unterschiedlichen Konzentrationen an R-Ibuprofenthioester (100, 10 und 1 ptm) eine Aktivierungshemmung von NF-κB (Spur 3-5) bewirkt. Spur 1 zeigt die unstimulierten, Spur 2 die stimulierten Kontrollzellen.

### Abb. 2: Hemmung der PGHS-2 durch R-Ibuprofenoyl-CoA.

Spur 1: 1.0 mM Ibuprofen, razemisch
Spur 2: 0.1 mM Ibuprofen, razemisch
Spur 3: 0.05 mM Ibuprofen, razemisch
Spur 4: 0.5 mM R-Ibuprofenoyl-CoA
Spur 5: 0.25 mM R-Ibuprofenoyl-CoA
Spur 6: 0.05 mM R-Ibuprofenoyl-CoA
Spur 7: Kontrolle, Medium + LPS

Inkubation von LPS-induzierten Monozyten (24 Std.). Die Abbildung zeigt, daß R-Ibuprofenoyl-CoA Thioester im Gegensatz zu razemischem Ibuprofen (R- und S-Ibuprofen) dosisabhängig zu einer Unterdrückung der Bildung von PGHS-2 (Cyclooxygenase-2) führt.
Methode nach: BRIDEAU, C., KARGMAN, S., LIU, S., DALLOB, A.L., EHRICH, E.W., RODGER, I.W. & CHAN, C.C. (1996). A human whole blood assay for clinical evaluation of biochemical efficacy of cyclooxygenase inhibitors. Inflamm. Res., 45, 68-74.

## Patentansprüche

1. Arzneimittel, enthaltend CoA-Thioester von Arylpropionsäuren, Arylessigsäuren oder Acetylsalicylaten oder R- oder/und S-Ibuprofen-CoA-Thioester.

2. Verwendung von CoA-Thioestern von Arylpropionsäuren, Arylessigsäuren oder Acetylsalicylaten oder R- oder/und S-Ibuprofenthioestern zur Herstellung eines Arzneimittels für die Indikationen akuter oder/und chronischer Schmerz, Entzündungen jedweder Genese und zur Bekämpfung aller anderen Symptome von entzündlichen Prozessen.

3. Verwendung von CoA-Thioestern von Arylpropionsäuren, Arylessigsäuren oder Acetylsalicylaten, R-Ibuprofen oder Derivaten der genannten Verbindungen oder Mischungen mit bis zu 49 % S-Ibuprofen zur Herstellung eines Arzneimittels zur Hemmung von Nf-κB-abhängigen Prozessen bei der Entstehung oder/und dem Wachstum von Tumoren oder allergischen Reaktionen.

4. Verwendung von CoA-Thioestern von Arylpropionsäuren, Arylessigsäuren oder Acetylsalicylaten, R-Ibuprofen oder Derivaten der genannten Verbindungen oder Mischungen mit bis zu 49 % S-Ibuprofen zur Herstellung eines Arzneimittels zur Hemmung von Nf-κB-abhängigen Prozessen bei Autoimmunerkrankungen.

## Claims

1. Pharmaceutical preparation containing CoA-thioesters of arylpropionic acids, arylacetic acids or acetylsalicylates or R-or/and S-ibuprofen-CoA-thioesters.

2. Use of CoA-thioesters of arylpropionic acids, arylacetic acids or acetylsalicylates or R- or/and S-ibuproten-thioesters to produce a pharmaceutical preparation for the indications of acute or/and chronic pain, inflammations of any genesis and to treat all other symptoms of inflammatory processes.

3. Use of CoA-thioesters of arylpropionic acids, arylacetic acids or acetylsalicylates, R-ibuprofen or derivatives of the said compounds or mixtures containing up to 49 % S-ibuprofen to produce a pharmaceutical preparation to inhibit Nf-κB-dependent processes in the formation or/and growth of tumours or allergic reactions.

4. Use of CoA-thioesters of arylpropionic acids, arylacetic acids or acetylsalicyates, R-ibuprofen or derivatives of said compounds or mixtures containing up to 49 % S-ibuprofen to produce a pharmaceutical preparation to inhibit Nf-κB-dependent processes in autoimmune diseases.

## Revendications

1. Médicament contenant des CoA-thioesters d'acides arylpropioniques, d'acides arylacétiques ou d'acétylsalicylates ou des CoA-thioesters de R- ou/et S-ibuprofène.

2. Utilisation de CoA-thioesters d'acides arylpropioniques, d'acides arylacétiques ou d'acétylsalicylates ou de CoA-thioesters de R- ou/et S-ibuprofène pour préparer un médicament destiné aux indications de douleurs aiguës ou/et chroniques, d'inflammations de toute origine et pour combattre tous les autres symptômes de processus inflammatoires.

3. Utilisation de CoA-thioesters d'acides arylpropioniques, d'acides arylacétiques ou d'acétylsalicylates, de R-ibuprofène ou de dérivés des composés précités ou de mélanges contenant jusqu'à 49% de S-ibuprofène pour préparer un médicament destiné à inhiber des processus Nf-κB-dépendants lors de la formation ou/et de la croissance de tumeurs ou de réactions allergiques.

4. Utilisation de CoA-thioesters d'acides arylpropioniques, d'acides arylacétiques ou d'acétylsalicylates, de R-ibuprofène ou de dérivés des composés précités ou de mélanges contenant jusqu'à 49% de S-ibuprofène pour préparer un médicament destiné à inhiber des processus Nf-κB-dépendants lors de maladies auto-immunes.
